(19) Europäisches Patentamt
European Patent Office
Office européen des brevets

(11) EP 1 674 495 A1

(12) EUROPEAN PATENT APPLICATION

(43) Date of publication:
**28.06.2006 Bulletin 2006/26**

(51) Int Cl.:
***C08G 59/18*** *(2006.01)* ***C08G 59/56*** *(2006.01)*
***C08G 59/62*** *(2006.01)*

(21) Application number: **04106911.3**

(22) Date of filing: **22.12.2004**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicants:
- **Huntsman Advanced Materials (Switzerland) GmbH
4057 Basel (CH)**
- **Huntsman Advanced Materials (Deutschland) GmbH
& Co. KG
59192 Bergkamen (DE)**

(72) Inventors:
- **Muller-Frischinger, Isabelle
68640 Riespach (FR)**
- **Gianini, Michel
2800 Delémont (CH)**
- **Volle, Jörg
59379 Selm-Bork (DE)**

(74) Representative: **Maué, Paul Georg
Solvias AG
Patents WKL-402.3.04
Klybeckstrasse 191
4002 Basel (CH)**

(54) **Coating system**

(57) Curable compositions comprising

a) an epoxy resin containing on average more than one epoxy group per molecule, and
b) as curing agent a hybrid hardener, whereby the said hardener is a blend of an amine selected from aliphatic, cycloaliphatic, araliphatic amines or imidazoline group-containing amidoamines based on mono-or polybasic acids or their adducts made from glycidyl compounds, which contain, on average, more than two reactive active hydrogens bound to amino nitrogen atoms per molecule, and a polyphenol (novolac) of the general formula (I) or a copolymer of different alkyl phenol units,

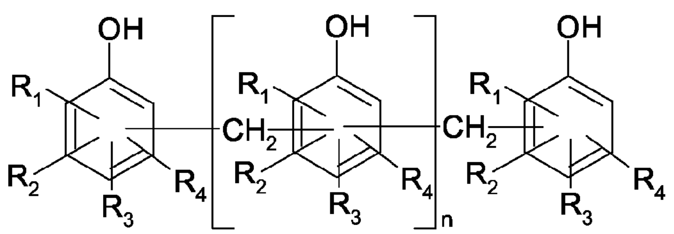
(I)

,

wherein in formula (I) $R_1$, $R_2$, $R_3$, $R_4$, are each independently of one another H, branched or unbranched alkyl radicals containing 1 to 15 carbon atoms, n is 1 to 15, and wherein the novolacs of formula (I) are used in amounts of at least 30% by weight, based on total hardener blend composed by the amine or imidazoline group-containing amidoamine or their adducts made from glycidyl compounds and the phenolic resin,
providing rapid setting protective coatings for adhesives and coatings in application fields civil engineering, marine, architectural and maintenance.



Printed by Jouve, 75001 PARIS (FR)

## Description

This invention relates to rapid setting coating systems, based on epoxy resins and, as hardeners, blends of amines and novolac resins, for use as a protective coating for metallic and mineral substrates.

Curable compositions, based on glycidyl compounds and traditional polyamines or polyamidoamines, are widely used for ambient-cure-temperature epoxy systems in adhesives and coating application fields like civil engineering, marine, architectural and maintenance.

However, amines with high reactivity and fast cure rate become more and more indispensable for certain applications that require rapid return to service or shorter manufacture time. For instance, the manufacturing of ships or the relining of water pipelines with rapid return to service are application areas, where the traditional epoxy/amine chemistry doesn't fulfill, at the moment, the requirements of rapid cure, especially at low temperature.

On another hand compounds, which are commonly used to accelerate epoxy/amine systems - like tertiary amines, acids, hydroxylamines, phenols (bisphenol A or bisphenol F) and Mannich bases (described, for instance, in WO 00/015687) - do not permit to achieve such fast curing properties at low temperature.

Therefore, the present invention deals with new hybrid hardeners based on amines and phenolic resins which, in combination with epoxies, exhibit very fast cure rate, even at temperatures close to 0°C. Depending on the amines or blends of amines mixed with the phenolic resin, the final epoxy compositions show surprisingly acceptable viscosity ranges, with exceptional fast cure speed. It was, as well, surprising to observe, that the general good chemical resistance of such cured epoxy systems was not altered by the introduction of phenolic resins into the epoxy/amine network. On the contrary, the chemical resistance of such systems was even improved toward chemicals like acetic acid 5% and 10%, compared to the neat epoxy/amine systems.

In the present work, aliphatic and araliphatic amines were used preferably. Optionally, imidazoline-group-containing polyamidoamines, based on mono- or polybasic acids, adducts thereof and Mannich bases can be used, as well.

Furthermore, with regard to devices such as tanks or pipelines, coming into contact with food or drinkable water, further important aspects, besides fast curing and long term durability of the coating, are the toxicological properties of the cured epoxy systems employed as protective coating. The migration of the constituents of the plastic materials and articles in food or potable water should not exceed certain limits. In this regard, "plastics" are understood as macromolecular compounds obtained by a process such as polymerization, polyaddition etc. Other substances or matter may be added to such macromolecular compounds. In any case, these substances should not migrate from the materials or articles into the foodstuffs, in quantities having a technological function in the final food. Considering for instance the UK legislation, the migration level of organic material for potable waterpipe (re)lining systems should not exceed 5 mg/liter (5 ppm) TOC (Total Organic Carbon).

As already mentioned, the present invention relates to rapid setting protective coating systems based on epoxy resins, useful, among other things, for internal (re)lining of pipes carrying potable water. Further possible applications include refurbishment of existing tanks, lines etc., which should return to service in a short time. In practice, this means that the curing should be achieved within a couple of hours, typically 2 to 5 hours, even at low temperatures, close to 0°C.

Accordingly, it is the object of this invention to provide fast epoxy systems which, while having a good cure speed at low temperature (as low as 3°C for waterpipe relining), are toxicologically safe and deliver low TOC values from migration tests.

Furthermore, such new hybrid hardeners could, as well, be useful for applications in which corrosion or chemical protection is required, such as tank lining. In this later case, conventional epoxy/amine systems exhibit poor resistance toward diluted acids. Only aromatic amines (e.g. diaminodiphenylmethane and derivatives) show a noticeable resistance toward diluted acids, like acetic acid. However, such aromatic amines might well be banned from the market in the future, due to toxicity reasons. Therefore, the new hybrid hardeners have been shown to improve, in an exceptional way, the chemical resistance toward diluted acids like acetic acid, depending on the type of amine(s) used. To that respect, MXDA based hybrid compositions show the best resistance toward such chemicals.

Another possible application area is marine, where many steel works on ships are exposed to salt, causing electrochemical corrosion and formation of rust. The anti-corrosive coating is generally applied to freshly sand-blasted steel and should be preferably cured and tacky-free after 24h, even at temperatures as low as 0°C. Such novel class of amine/phenolic resin compositions offers both, rapid cure and improved resistance to corrosion, as can be seen from salt spray tests, performed according to the DIN 53167 method. Compared to hardeners like phenalkamines, which are as well suitable for low temperature cure, such hybrid systems are much more rapid, while ensuring good corrosion resistance. Last but not least, the new hybrid hardeners are much less colored than phenalkamines, with the exception of natural alkylphenol cardanols having unsaturated double bonds and being more or less brownish colored raw materials.

This invention, therefore, relates to curable compositions comprising

a) an epoxy resin containing on average more than one epoxy group per molecule, and

b) as curing agent a hybrid hardener, whereby the said hardener is a blend of an amine selected from aliphatic, cycloaliphatic, araliphatic amines or imidazoline group-containing amidoamines based on mono-or polybasic acids or their adducts made from glycidyl compounds, which contain, on average, more than two reactive active hydrogens bound to amino nitrogen atoms per molecule, and a polyphenol (novolac) of the general formula (I) or a copolymer of different alkyl phenol units,

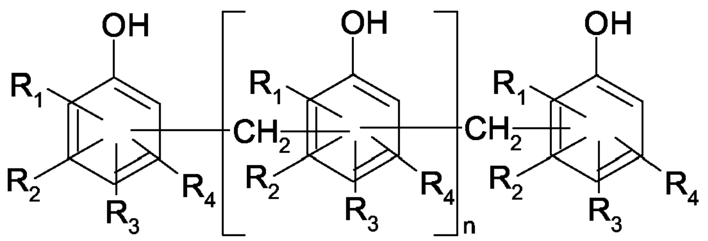

(I)

,

wherein in formula (I) $R_1$, $R_2$, $R_3$, $R_4$ are each independently of one another H, branched or unbranched alkyl radicals containing 1 to 15 carbon atoms, n is 1 to 15, and wherein the novolacs of formula (I) are used in amounts of at least 30% by weight, based on total hardener blend composed by the amine or imidazoline group-containing amidoamine or their adducts made from glycidyl compounds and the phenolic resin.

The novolac of formula (I) should preferably not exceed 45% by weight based on total hardener blend in order to get a liquid hardener composition at ambient conditions. In the case of very high hardener viscosities, it is desirable to add a solvent to the hardener blend in order to reduce the viscosity of the final formulation to make said formulation spray or brush applicable.

The novolacs used in this invention can be prepared according to well-known processes, on reacting formaldehyde or paraformaldehyde with phenolic compound(s) - such as phenol, cresol, xylenol, alkylphenol and the like - on using, if required, a catalyst such as oxalic acid, and on removing the water formed by the reaction. The novolacs can be made from one or a a mixture of two or more than two different phenolic compounds.

Such products are described, inter alia, in Houben-Weyl, 4th edition, Methoden der Organischen Chemie, Vol. E 20, Makromolekulare Stoffe, Part 3, pages 1800-1806.

To prepare these products, the phenolic compound(s) as well as catalytic amounts of oxalic acid are generally placed in a vessel - with or without solvent or water - and formaldehyde, but preferably paraformaldehyde, is added in portions. The volatile components are then removed by distillation under reduced pressure.

The novolacs prepared in this way are statistical compositions, wherein the repeating factor n is preferably 1 to 12, more preferably 1 to 10, most preferably 1 to 8, depending on the polydispersity index of the polymer. It is well-known that a narrow distribution of polymer (polymer index Ip ~ 1.0) leads to polymer solutions with lower viscosity ranges. Therefore, in order to reduce as much as possible the viscosity of the final system, a polydispersity index Ip around 1 is preferred and n is 1 to 6. A good example of commercially available phenol novolac is Supraplast® 3616 from Südwest-Chemie, whose polydispersity index Mw/Mn lies around 1.24 . The average n value can be easily influenced on using a suitable excess of phenolic component(s) with respect to the amount of (para)formaldehyde.

Preferred novolacs are those, wherein in formula (I) $R_1$, $R_2$, $R_3$, $R_4$ are either H, or one or two of the radicals $R_1$ to $R_4$ are the radical $-CH_3$, or one of the radicals $R_1$ to $R_4$ is a tert-butyl radical, or one of the radicals $R_1$ to $R_4$ is a long-chain branched or unbranched alkyl radical containing 8 to 15 carbon atoms. However the novolac resin used should not be too viscous with low molecular weight and narrow molecular weight distribution with a polydispersity index approaching 1 like in the case of Supraplast 3616.

As a copolymer of different alkylphenol units is meant, that a novolac of the general formula (I) above can contain different types of units of alkylphenols with regard to their individual radicals $R_1$ to $R_4$ simply by making the novolac from a mixture of at least two different phenolic compounds. Otherwise the same definitions for the radicals $R_1$ to $R_4$ and repeating factor n is valid as given for compounds of formula (I).

The epoxy compounds, additionally used according to this invention for the preparation of the curable compositions, are commercially available products containing more than one epoxy group per molecule and are derived from mono- and polyvalent, mono- and/or polynuclear phenols, in particular bisphenols, and from novolacs. An extensive enumeration of these di- and polyphenols is to be found in the compendium "Epoxidverbindungen und Epoxidharze" by A. M. Paquin, Springer Verlag, Berlin, 1958, chapter IV, and in Lee & Neville, "Handbook of Epoxy Resins", 1967, chapter 2, pages 257-307. It is also possible to use compositions of two or more epoxy resins.

It is preferred to use liquid glycidyl compounds based on bisphenol A, bisphenol F and polyfunctional aromatic

or aliphatic epoxy resins or eventually solid resins, usually of type 1, where solvent is needed to dissolve the epoxy resin and to reduce the viscosity in such a way that the product can be sprayed, as it is the case in marine applications.

Blends of epoxy resins with so-called reactive diluents, e.g. monoglycidyl ethers of phenols or of mono- or difunctional aliphatic or cycloaliphatic alcohols, can be used, as well.

These reactive diluents primarily serve to reduce the viscosity and should only be used in small amounts, as they adversely affect the end-properties of the thermoset. The epoxy resins mentioned as examples can be used both for the curable compositions and for the preparation of the amine-epoxy adducts, that may be blended with the phenolic resin.

The amines, which are blended with the phenolic resins and cured with the epoxy resins according to this invention are aliphatic, cycloaliphatic, or araliphatic amines, or imidazoline group-containing amidoamines based on mono-or polybasic acids or their adducts made from glycidyl compounds, which contain, on average, more than two reactive active hydrogens bound to amino nitrogen atoms per molecule. These compounds are part of the general state of the art and are described, inter alia, in Lee & Neville, "Handbook of Epoxy Resins", MC Graw Hill Book Company, 1987, chapter 6-1 to 10-19.

The amines used according to this invention are aliphatic, cycloaliphatic or araliphatic amines like: 1,2-diaminoethane (ethylenediamine (EDA)); 1,2-propanediamine; 1,3-propanediamine; 1,4-diaminobutane; 2,2-dimethyl-1,3-propanediamine (neopentanediamine); diethylaminopropylamine (DEAPA); 2-methyl-1,5-diaminopentane; 1,3-diaminopentane; 2,2,4-Trimethyl-1,6-diaminohexane; 2,4,4-Trimethyl-1,6-diaminohexane and mixtures thereof (TMD); 1-amino-3-aminomethyl-3,5,5-trimethylcyclohexane; 1,3-bis(aminomethyl)-cyclohexane; 1,2-bis(aminomethyl)cyclohexane; hexamethylenediamine (HMD); 1,2- and 1,4-Diaminocyclohexane (1,2-DACH and 1,4-DACH) ; bis(4-aminocyclohexyl) methane; bis(4-amino-3-methylcyclohexyl)methane; diethylenetriamine (DETA); 4-azaheptane-1,7-diamine; 1,11-diamino-3,6,9-trioxundecane; 1,8-diamino-3,6-dioxaoctane; 1,5-diamino-methyl-3-azapentane; 1,10-diamino-4,7-dioxadecane; Bis(3-aminopropyl)amine; 1,13-diamino-4,7-10 trioxatridecane; 4-aminomethyl-1,8-diaminooctane; 2-butyl-2-ethyl-1,5-diaminopentane; N,N-Bis-(3-aminopropyl)methylamine; triethylenetetramine (TETA); tetraethylenepentamine (TEPA); pentaethylenehexamine (PEHA); Bis(4-amino-3-methylcyclohexyl)methane; m-xylylenediamine (MXDA); 5-(aminomethyl)bicyclo[[2.2.1]hept-2-yl]methylamine (NBDA norbornanediamine); dimethyldipropylenetriamine; dimethylaminopropyl-aminopropylamine (DMAPAPA); 3-Aminomethyl-3,5,5-trimethylcyclohexylamine (or isophoronediamine (IPD)); diaminodicyclohexylmethane (PACM) ; dimethyldiaminodicyclohexylmethane (Laromin C260); 2,2-Bis (4-aminocyclohexyl)propane; bis aminomethyl-dicyclopentadiene (tricyclodecyldiamine (TCD)); imidazoline-group-containing polyaminoamides derived from aliphatic polyethylene polyamines and dimerized or trimerized fatty acids and adducts thereof made from glycidyl compounds.

Further, polyoxyalkylene polyamines, known as Jeffamine® , from Huntsman like D-230, D-400, D- 2000, T-403, T-3000, T-5000, ED-600, ED-900, EDR148, and polyiminoalkylene polyamines, known as Polymin® , can be used, as well, to be blended with phenolic resins within the frame of the present invention.

Further suitable polyamines are 1,14-diamino-4, 11-dioxatetradecane; dipropylenetriamine; 2-methyl-1,5-pentanediamine; N,N'-dicyclohexyl-1,6-hexanediamine; N,N'-dimethyl-1,3-diaminopropane; N,N'-diethyl-1,3-diaminopropane; N,N-dimethyl-1,3-diaminopropane; secondary polyoxypropylenedi- and triamine; 2,5-diamino-2,5-dimethylhexane; bis-(amino-methyl)tricyclopentadiene; 1,8-Diamino-p-menthane; Bis- (4-amino-3,5-dimethylcyclohexyl)methane; 1,3-Bis(aminomethyl)cyclohexane (1,3-BAC); dipentylamine. N-2-(aminoethyl)piperazine (N-AEP); N-3-(aminopropyl) piperazine; piperazine.

Preferred amines are selected from MXDA, IPD, TMD and 1,3-BAC.

Finally, mixtures from several of the above mentioned amines are, as well, possible.

Furthermore, if the application is connected with foodstuff - as it is the case of drinking water pipelines - the aliphatic, cycloaliphatic or araliphatic amines, entering in the composition of the hybrid hardeners, should not infringe the regional regulations for plastics in contact with food or drinkable water. In Europe for instance, the polyamines, as well as all other components used in the new hybrid systems, have be found in the "positive list" published by the "Commission of the European Communities" in "Directive 2002/72/EC" of 6th August 2002 and "Directive 2004/19/EC" of 1st March 2004. Accordingly, amines found in said first draft "positive list" of the EU legislation - as it is the case of mixtures of 1,6-diamino-2,2,4-trimethylhexane and 1,6-diamino-2,4,4- trimethylhexane (TMD), xylylenediamine (MXDA), isophoronediamine (IPD) and blends thereof - can be used for potable water pipelines.

Hybrid hardeners and epoxy compounds are preferably used in about equivalent amounts, i.e. based on active hydrogens bound to amino nitrogen atoms and reactive epoxy groups. However, it is also possible to use the hybrid hardener or the glycidyl component in more or in less than the equivalent amount. The amounts used depend on the desired final properties of the reaction product.

Standard accelerators for epoxy/amine reaction can be used in addition to the new amine/polyphenol hybrid hardeners. For instance, a catalyst like salicylic acid could be added to the system to accelerate even more the cure speed.

Depending on the field of application and on the end use requirements, it is also possible to add to the novel hybrid epoxy compositions inorganic and/or organic additives, such as finely divided sands, talcum, silicic acid, alumina, metal or metal oxide compounds in chips or powder form, thixotropic agents, fibrous substances, pigments, flame-retarding agents, solvents, colorants, plasticisers, bitumen. Obviously, only those of said additives may be employed, provided that they have no resulting adverse effects on drinking water or on food quality.

As mentioned before, the novolacs according to general formulae (I) are used in amounts of at least 30%, preferably more than 30% by weight based on amine, but no more than 45% by weight based on amine. The quantity of phenolic resin depends principally on the type of amine or mixture of amines as well on the type of phenolic resin used to prepare the hybrid hardener and on the targeted viscosity/properties for a given application. To that respect, the viscosity of the hybrid hardeners should be preferably lower than 20 000 mPa•s at ambient temperatures (ca. 25°C), since additional solvent is needed to fix the viscosity at lower viscosity ranges in order to spray apply the formulation and since it is wished to have formulation with high solid content (> 80%) for ecological reasons. Standard solvents, like xylene/butanol mixtures or pure alcohols are commonly used. But organic solvents can be used only in certain cases, as they are not recommended, for instance, in potable water pipe re(lining) or wine tank lining applications.

Similar compositions to those of our invention are known from WO 99/29757; however the phenol novolacs, therein, are used in amounts being typical for acceleration, with weight percentage of 1 to 25%, based on amine hardener. In the case of the present invention, it is more appropriate to speak of amine/novolac hybrids, as it has surprisingly been found that the most efficient ratio between amine/novolac, in terms of curing rate and chemical resistance, is between 70/30 and 55/45. Surprisingly, the amine/novolac blend remains liquid, even at ratios around 60/40. For example if Supraplast 3616 is used as phenolic resin, the best ratio of the amine/novolac blends is found to be 60/40 in the case of the amines trimethylhexamethylenediamine (TMD) or m-xylylenediamine (MXDA). As mentioned before, the proportions of amine/polyphenol can be varied, depending on the desired properties in terms of viscosity, curing rate, chemical resistance and corrosion protection. It was surprisingly observed, that the chemical resistance toward aggressive chemicals like acetic acid 5 and 10 wt% could be improved, using a modification level of polyphenol in the amine comprising a ratio of between 30 and 45 wt% of the polyphenol in the blend. It was not expected, at this stage, that the reduced network density, due to the modification of the amine with polyphenol, would affect positively the resistance toward acids like acetic acid.

Examples

A) Cure properties of hybrid hardeners based on amine and polyphenol Supraplast 3616

The following hybrid hardeners have been prepared by dissolving the novolac resin Supraplast 3616 in different amines or amine mixtures at the temperature of 80°C; the characteristics of hybrid hardener are given below in table 1.

Table 1:Hybrid hardener compositions with different ratios of amine and polyphenolic resin

| Blend | A | B | C |
|---|---|---|---|
| MXDA 1) | 30.0 | 59.0 | ----- |
| TMD 2) | 30.0 | ----- | 60.0 |
| Novolac Supraplast 3616 3) | 40.0 | 41.0 | 40.0 |
| Viscosity hardener at 25°C 4) | 7430 | 7160 | 8550 |

1) MXDA = m-xylylenediamine; 2) TMD = Trimethylhexamethylenediamine, 3) Novolac resin Supraplast 3616 purchased at Süd-West-Chemie GmbH Neu-Ulm with following characteristics Mn=341, Mw=474, Ip= 1.39 determined by using GPC-RI: Columns: 3 x Mixed-C; eluant: THF at 1ml/min, Polystyrene calibration; 4) Viscosity of amine/novolac blend was determined at 25°C using a CAP 2000 viscosimeter at 50 rpm and with cone 3.

The table 2 below gives the cure properties of the different epoxy systems comprising different blends of amine/novolac resin cured at both temperatures 0°C and 5°C.

Table 2: Cure properties of the hybrid hardeners amine/polyphenolic resin Supraplast 3616

| Formulation | 1 | 2 | 3 |
|---|---|---|---|
| Epoxy resin 1) | 73.86 | 75.03 | 72.20 |
| amine/novolac blend A) 2) | 23.14 | --- | --- |
| amine/novolac blend B) 2) | --- | 24.97 | --- |

Table continued

| Formulation | 1 | 2 | 3 |
|---|---|---|---|
| amine/novolac blend C) 2) | --- | --- | 27.80 |
| Viscosity of formulation at 25°C 3) | 9400 | 9800 | 11000 |
| Curing at 0°C (hours) 4) | 3.5 | 3.5 | 3.0 |
| Dust free at 0°C (hours) 5) | 4.5 | 4.0 | 4.0 |
| Curing at 5°C (hours) 4) | 3.0 | 2.5 | 3.0 |
| Dust free at 5°C (hours) 5) | 3.5 | 2.5 | 2.0 |
| 1) PY302-2 with Epoxy equivalent weight of EEW 173 in wt%; 2) in wt%; 3) Viscosity of the formulation was determined at 25°C using a CAP 2000 viscosimeter with cone 3 at 50 rpm (ISO 3219); 4)5) the cure times were measured on Landolt equipment using glass sheets coated with the above formulations. To determine the full cure, a needle is continuously moving forward on the coated glass during exactly 24h; the full cure is determined by measuring the distance/time where the needle, penetrating the film, comes out from the film. To detemine dust free time, sand is continuously added to the coating surface; the dust free time is measured by removing the sand from the coating surface and measuring the distance/time where sand sticks on the coating surface. | | | |

The results given in table 2 show excellent cure times for both measurement methods full cure and dust free even at 0°C, which is very exceptional for epoxy systems.

The table 3 below gives the hardness in Shore D in function of the cure times at different cure temperatures and relative air humidity conditions for formulation 3 as listed in table 2.

Table 3: Hardness in Shore D of coatings made with the above hybrid hardener C (in table 1) in function of the cure time and for different relative air humidity conditions

| Shore D values 1)for formulation | 3 |
|---|---|
| after 1 day (d) at 0°C | 70 |
| 2 d at 0°C | 70 |
| 7 d at 0°C | 75 |
| after 1 day (d) at 5°C | 70 |
| 2 d at 5°C | 80 |
| 7 d at 5°C | 80 |
| after 1 day (d) at 23°C/50% rel. air humidity | 80 |
| 2 d at 23°C/50% rel. air humidity | 80 |
| 7d at 23°C/50% rel. air humidity | 80 |
| after 1 day (d) at 23°C/100% rel. air humidity | 70 |
| 2 d at 23°C/100% rel. air humidity | 70 |
| 7d at 23°C/100% rel.air humidity | 75 |
| 1) Shore hardness was measured using a 4 mm thick sample following the Shore D hardness test ISO 868 & DIN 53505 (method A/D) | |

The results of table 3 shows very high Shore D hardness obtained after one day cure even at 0°C, which shows the exceptional fast cure rate of such system.

B) Migration tests of hybrid hardeners amine/novolac resin for use in water re(lining) systems

The TOC (Total Organic Carbon) results were obtained using, for the preparation of the sample, an internal

method of cure and simulation of pipe cleaning. They were obtained using following method of preparation:
The bottom of a beaker glass having a diameter of 8 cm is cleaned with acetone and deionised water. A 1 mm thick material is casted on the bottom of the becher glass; the material is allowed to cure during 3 hours at 3°C and after this time the surface of the material is cleaned with a stream of water during 1 hour to simulate the cleaning of the pipe. The material is then extracted with 200 ml deionised water during 24 hours and the extract is analysed for its TOC content (using method Norm ISO 8245:1999: dosage of carbon organic total (COT) and the organic carbon dissolved (COD)).
The following table 4 gives TOC values for formulations 1 to 3 given in table 2.

Table 4: Migration tests TOC results of hybrid hardeners cured with araldite PY 302-2

| Formulation | 1 | 2 | 3 |
|---|---|---|---|
| TOC [ppm] | 0.2 | 0.6 | 0.6 |
| Note: The actual measured values are between 0.4 and 0.8. Although the deionisized water itself has already a TOC of 0.2 ppm. | | | |

C) Chemical resistance of hybrid hardeners amine/novolac resin combined with epoxy resin

The chemical resistance was tested on coatings applied approximately 500 microns thick on sand-blasted steel panels, which were cured for 10 days at 23°C 50% rh.
The chemical resistances of such hybrid hardeners amine/phenolic resin were compared to these of unmodified amines for instance TMD or MXDA and also to these of amine/novolac resin mixtures being at the superior limit of the compositions claimed in the patent WO 99/29757 for instance at the ratio amine/novolac resin 75/25.
First the chemical resistances of pure amines like TMD and MXDA are given respectively in table 5 and 6. Both amines don't show at all resistance toward acetic acid 5 and 10 wt%, the films being destroyed in less than 3 days once in contact with such aggressive chemicals.

Table 5: Chemical resistance of the neat system MXDA/Araldite GY250 (unmodified system)

| Epoxid / Hardener | Araldite GY250: 84.56 parts / MXDA : 15.44 parts | | | | | | | | | |
|---|---|---|---|---|---|---|---|---|---|---|
| Duration (d, w, m) [1] | 1 d | 3 d | 1 w | 2 w | 1 m | 2 m | 3 m | 4 m | 5 m | 6 m |
| $C_6H_4(CH_3)_2$ | ■ | ■ | ■ | ■ | ■ | ■ | ■ | | | |
| $C_2H_5OH$ 95% | ■ | ■ | ■ | ■ | ■ | ■ | ■ | | | |
| $CH_3COOH$ 10% | ⌘ | ⌘ | ❒ | ❒ | ❒ | ❒ | ❒ | | | |
| $CH_3COOH$ 5% | ❒ | ❒ | ❒ | ❒ | ❒ | ❒ | ❒ | | | |

For tables 5 to 10:

[1] d, w, m = days, weeks ,months

coating surface either ■ = resistant to, ⌘ = attacked by, or ❒ =destroyed by the chemical

Table 6: Chemical resistance of the neat system MXDA/Araldite GY250 (unmodified system)

| Epoxid / Hardener | Araldite GY250: 82.32 parts / TMD : 17.68 parts | | | | | | | | | |
|---|---|---|---|---|---|---|---|---|---|---|
| Duration (d, w, m) 1) | 1 d | 3 d | 1 w | 2 w | 1 m | 2 m | 3 m | 4 m | 5 m | 6 m |
| $C_6H_4(CH_3)_2$ | ■ | ■ | ■ | ■ | ■ | ■ | ■ | | | |
| $C_2H_5OH$ 95% | ■ | ■ | ■ | ⌘ | ⌘ | ⌘ | ⌘ | | | |
| $CH_3COOH$ 10% | ⌘ | ⌘ | ❐ | ❐ | ❐ | ❐ | ❐ | | | |
| $CH_3COOH$ 5% | ⌘ | ⌘ | ⌘ | ⌘ | ⌘ | ❐ | ❐ | | | |

The chemical resistances of coatings made with the above amines containing Supraplast 3616 of about 25wt%, the superior limit that is mentioned in the patent WO 99/29757, are given in table 7 and 8. The chemical resistance is improved a little bit in both cases TMD and MXDA, but the coatings are attacked or destroyed in a short time in the case of the aggressive chemical 10wt% acetic acid.

Table 7: Chemical resistance of MXDA containing 25 wt% novolac resin Supraplast 3616

| Epoxid / Hardener | Araldite GY250: 80.43 p. / MXDA/Supraplast 75/25: 19.57 p. | | | | | | | | | |
|---|---|---|---|---|---|---|---|---|---|---|
| Duration (d, w, m) 1) | 1 d | 3 d | 1 w | 2 w | 1 m | 2 m | 3 m | 4 m | 5 m | 6 m |
| $C_6H_4(CH_3)_2$ | ■ | ■ | ■ | ■ | ■ | ■ | ■ | | | |
| $C_2H_5OH$ 95% | ■ | ■ | ■ | ■ | ■ | ■ | ■ | | | |
| $CH_3COOH$ 10% | ■ | ■ | ⌘ | ⌘ | ⌘ | ❐ | ❐ | | | |
| $CH_3COOH$ 5% | ■ | ■ | ■ | ■ | ■ | ■ | ■ | | | |

Table 8: Chemical resistance of TMD containing 25 wt% novolac resin Supraplast 3616

| Epoxid / Hardener | Araldite GY250: 77.74 p. / TMD/Supraplast 75/25: 22.26 p. | | | | | | | | | |
|---|---|---|---|---|---|---|---|---|---|---|
| Duration (d, w, m) 1) | 1 d | 3 d | 1 w | 2 w | 1 m | 2 m | 3 m | 4 m | 5 m | 6 m |
| $C_6H_4(CH_3)_2$ | ■ | ■ | ■ | ■ | ■ | ■ | ■ | | | |
| $C_2H_5OH$ 95% | ⌘ | ⌘ | ⌘ | ⌘ | ⌘ | ⌘ | ⌘ | | | |
| $CH_3COOH$ 10% | ⌘ | ⌘ | ⌘ | ❐ | ❐ | ❐ | ❐ | | | |
| $CH_3COOH$ 5% | ⌘ | ⌘ | ⌘ | ⌘ | ⌘ | ⌘ | ⌘ | | | |

The chemical resistance of the new hybrid compositions containing high amounts of Supraplast 3616 of almost 40 wt% are given for both cases MXDA and TMD respectively in table 9 and 10. The resistance of the coatings are improved especially in the case of TMD hardener for which the resistance toward acetic 5wt% pass 6 months. Also the time of resistance toward the very aggressive chemical acetic acid 10wt% is prolonged in both cases of a couple of months.

Table 9: Chemical resistance of MXDA modified with 40 wt% novolac resin Supraplast 3616

| Epoxid / Hardener | Araldite GY250: 76.36 p. / MXDA/Supraplast 59/41: 23.64 p. | | | | | | | | | |
|---|---|---|---|---|---|---|---|---|---|---|
| Duration (d, w, m) [1)] | 1 d | 3 d | 1 w | 2 w | 1 m | 2 m | 3 m | 4 m | 5 m | 6 m |
| $C_6H_4(CH_3)_2$ | ■ | ■ | ■ | ■ | ■ | ■ | ■ | ■ | ■ | ■ |
| $C_2H_5OH$ 95% | ■ | ■ | ■ | ■ | ■ | ■ | ■ | ■ | ■ | ■ |
| $CH_3COOH$ 10% | ■ | ■ | ■ | ■ | ■ | ■ | ⌘ | ❐ | ❐ | ❐ |
| $CH_3COOH$ 5% | ■ | ■ | ■ | ■ | ■ | ■ | ■ | ■ | ■ | ■ |

Table 10: Chemical resistance of TMD modified with 40 wt% novolac resin Supraplast 3616

| Epoxid / Hardener | Araldite GY250: 73.64 p. / TMD/Supraplast 60/40: 26.36 p. | | | | | | | | | |
|---|---|---|---|---|---|---|---|---|---|---|
| Duration (d, w, m) [1)] | 1 d | 3 d | 1 w | 2 w | 1 m | 2 m | 3 m | 4 m | 5 m | 6 m |
| $C_6H_4(CH_3)_2$ | ■ | ■ | ■ | ■ | ■ | ■ | ■ | ■ | ■ | ■ |
| $C_2H_5OH$ 95% | ■ | ■ | ■ | ■ | ■ | ⌘ | ⌘ | ⌘ | ⌘ | ⌘ |
| $CH_3COOH$ 10% | ■ | ■ | ■ | ■ | ■ | ■ | ⌘ | ⌘ | ❐ | ❐ |
| $CH_3COOH$ 5% | ■ | ■ | ■ | ■ | ■ | ■ | ■ | ■ | ■ | ■ |

D) Corrosion resistance of hybrid system based on TMD/Supraplast 3616

The corrosion resistance was measured following DIN 35167, the salt spray test. The hybrid hardener C [TMD/Supraplast 60/40] of table 1 was formulated as an anticorrosive primer given in table 11 and applied with spray gun on sand blasted steel panels 100mm x 70 mm at thicknesses of 160 µm and 80 µm. The coated panels were allowed to cure during 7 days at 23°C /50% rh. After this time the coated panels were scribed in X-shape with the Scratch Stylus 463 from Erichsen, each leg being approximately 5 cm in length. The panels were then exposed to salt spray during different times for instance 500 h, 1000 h, 2000 h and 4000 h.

Table 11: Anticorrosive primer formulation using copolymers of phenol and alkyl phenol

| Primer formulation epoxy/hybrid hardener | Parts by weight |
|---|---|
| GY 250 | 85.1 |
| Luvotix P 25 X (thickener) | 8.1 |
| ZP 10 (Zinc phosphate) | 22.5 |
| Talc 10 MO | 75.3 |
| Iron oxide 130 | 14.85 |
| $BaSO_4$ EWO S | 30.1 |
| Xylene-butanol 4:1 | 69.5 |
| Hardener C | 30.4 |
| - Luvotix P 25 X is a thixotropic agent (Lehmann&Voss&Co); Zinc phosphate ZP 10 (Heubach GmbH)<br>-Talc 10 MO (Talc de Luzenac France); Barium sulfate EWO® -S (Sachtleben Chemie GmbH) | |

Table continued

| |
|---|
| -iron oxide red 130 (BAYER); - Hardener C (see table 1) |

The results of the corrosion test in the case of the hybrid hardener C are given in table 12. The value $W_A$ in formula I depends on the undercoat corrosion area created during the exposure time. As higher this value becomes, as worse is the corrosion resistance of the coating. In the present case, minimal undercoat corrosion was observed during an exposure time of almost 4000 h (hours).

$$\text{Formula (I):} \qquad W_A = \frac{A_1 - A_0}{2} \cdot \frac{1}{L}$$

$A_1$ = Total surface of the under corrosion zone in $mm^2$
$A_0$ = Surface of the scrubbed line in $mm^2$
L = Length of the scrubbed line in mm

<u>Table 12:</u> $W_A$ value obtained at different corrosion times for the hybrid hardener C in table 1

| System / Exposure time | 500 h | 1000 h | 2000 h | 4000 h |
|---|---|---|---|---|
| TMD/ Supraplast [60/40] 80 μm | 0 | 7 | 8 | 16 |
| TMD/ Supraplast [60/40] 160 μm | 1 | 13 | 15 | 16 |
| TMD/ Supraplast [60/40] 80 μm corroded surface $mm^2$ | 0 | 153 | 177 | 337 |
| TMD/ Supraplast [60/40] 160 μm corroded surface $mm^2$ | 30 | 261 | 310 | 334 |
| $A_0$ = Surface of the scrubbed line in $mm^2$ = 10 x 1 $mm^2$<br>L = Length of the scrubbed line in mm = 10 mm | | | | |

The coatings made with such hybrid hardener based on TMD show subsequently excellent resistance to corrosion with minimal undercoat corrosion and can for instance be further used in marine primer formulation.

**Claims**

**1.** A curable composition comprising

a) an epoxy resin containing on average more than one epoxy group per molecule, and
b) as curing agent a hybrid hardener, whereby the said hardener is a blend of an amine selected from aliphatic, cycloaliphatic, araliphatic amines or imidazoline group-containing amidoamines based on mono-or polybasic acids or their adducts made from glycidyl compounds, which contain, on average, more than two reactive active hydrogens bound to amino nitrogen atoms per molecule, and a polyphenol (novolac) of the general formula (I) or a copolymer of different alkyl phenol units,

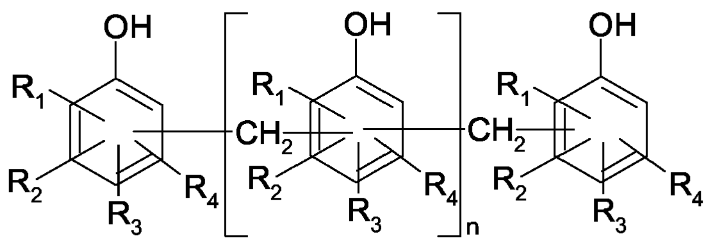

(I)

,

wherein in formula (I) $R_1$, $R_2$, $R_3$, $R_4$, are each independently of one another H, branched or unbranched alkyl radicals containing 1 to 15 carbon atoms, n is 1 to 15, and wherein the novolacs of formula (I) are used in amounts of at least 30% by weight, based on total hardener blend composed by the amine or imidazoline group-containing amidoamine or their adducts made from glycidyl compounds and the phenolic resin.

**2.** A curable composition according to claim 1, wherein the repeating factor n for formula (I) is preferably n = 1 -12.

**3.** A curable composition according to claim 1, wherein in formula (I) $R_1$, $R_2$, $R_3$, $R_4$ is H.

**4.** A curable composition according to claim 1, wherein in formula (I) one or two of the radicals $R_1$ to $R_4$ are $-CH_3$ radicals.

**5.** A curable composition according to claim 1, wherein in formula (I) one of the radicals $R_1$ to $R_4$ is a tert-butyl radical.

**6.** A curable composition according to claim 1, wherein in formula (I) one of the radicals $R_1$ to $R_4$ is a branched or unbranched alkyl radical containing 8 to 15 carbon atoms.

**7.** A curable composition according to claim 1, wherein the novolacs of formula (I) are used in amounts of 30 to 45 wt% based on total hardener blend composed by the amine or imidazoline group-containing amidoamine or their adducts made from glycidyl compounds and the phenolic resin.

**8.** A curable composition according to claim 1, wherein the amine is selected from aliphatic and araliphatic amines.

**9.** A curable composition according to claim 8, wherein the amine is selected from trimethylehexamethylenediamine (TMD), m-xylylenediamine (MXDA), isophoronediamine (IPD), and 1,3-bis(aminomethyl)cyclohexane (1,3-BAC).

**10.** A curable composition according to claim 1, wherein the epoxy resin is selected from bisphenol A, bisphenol F and polyfunctional aromatic or aliphatic epoxy resins.

**11.** A curable composition according to any one of claims 1 to 10 additional comprising inorganic and/or organic additives selected from finely divided sands, talcum, silicic acid, alumina, metal or metal oxide compounds in chips or powder form, thixotropic agents, fibrous substances, colorants, pigments, flame-retarding agents, solvents, water, colorants, plasticisers, bitumen, conventional accelerators, UV stabilisers, fillers.

**12.** Use of a composition according to any one of claims 1 to 11 for providing protective coatings for adhesives and coatings in application fields civil engineering, marine, architectural and maintenance.

European Patent Office

# EUROPEAN SEARCH REPORT

Application Number
EP 04 10 6911

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 99/29757 A (CIBA SPEZIALITAETENCHEMIE BERGKAMEN GMBH; SCHERZER, WOLFGANG; VOLLE, J) 17 June 1999 (1999-06-17)<br>* the whole document * | 1-12 | C08G59/18<br>C08G59/56<br>C08G59/62 |
| X | EP 0 365 479 A (CIBA-GEIGY AG)<br>25 April 1990 (1990-04-25)<br>* claim 1; examples; table 1 * | 1-6,8, 10-12 | |
| A | * page 5, lines 26,27 * | 7 | |
| A | * page 5, lines 1-13 * | 9 | |
| X | EP 0 266 306 A (CIBA-GEIGY AG)<br>4 May 1988 (1988-05-04)<br>* page 5, lines 9-11; examples *<br>* page 5, lines 30-34 * | 1-6,8-12 | |
| A | * page 5, lines 56-61 * | 7 | |

TECHNICAL FIELDS SEARCHED (Int.Cl.7)

C08G

The present search report has been drawn up for all claims

1

| Place of search | Date of completion of the search | Examiner |
|---|---|---|
| Munich | 18 May 2005 | Marquis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 10 6911

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2005

| Patent document cited in search report | | Publication date | Patent family member(s) | | | Publication date |
|---|---|---|---|---|---|---|
| WO 9929757 | A | 17-06-1999 | BR | 9813419 | A | 10-10-2000 |
| | | | CN | 1113074 | C | 02-07-2003 |
| | | | DE | 69811729 | D1 | 03-04-2003 |
| | | | DE | 69811729 | T2 | 14-08-2003 |
| | | | WO | 9929757 | A1 | 17-06-1999 |
| | | | EP | 1040150 | A1 | 04-10-2000 |
| | | | ES | 2191366 | T3 | 01-09-2003 |
| | | | JP | 2001525469 | T | 11-12-2001 |
| | | | US | 6649729 | B1 | 18-11-2003 |
| EP 0365479 | A | 25-04-1990 | AT | 131842 | T | 15-01-1996 |
| | | | CA | 2000761 | A1 | 18-04-1990 |
| | | | DE | 68925179 | D1 | 01-02-1996 |
| | | | DE | 68925179 | T2 | 22-08-1996 |
| | | | EP | 0365479 | A2 | 25-04-1990 |
| | | | ES | 2081309 | T3 | 01-03-1996 |
| | | | HK | 1004274 | A1 | 20-11-1998 |
| | | | JP | 2158618 | A | 19-06-1990 |
| | | | JP | 2818954 | B2 | 30-10-1998 |
| | | | US | 4977214 | A | 11-12-1990 |
| | | | US | 5021513 | A | 04-06-1991 |
| EP 0266306 | A | 04-05-1988 | AT | 94185 | T | 15-09-1993 |
| | | | CA | 1293340 | C | 17-12-1991 |
| | | | DE | 3787343 | D1 | 14-10-1993 |
| | | | EP | 0266306 | A2 | 04-05-1988 |
| | | | ES | 2059405 | T3 | 16-11-1994 |
| | | | HK | 1004557 | A1 | 27-11-1998 |
| | | | JP | 2092997 | C | 18-09-1996 |
| | | | JP | 7119274 | B | 20-12-1995 |
| | | | JP | 63117032 | A | 21-05-1988 |
| | | | US | 4866133 | A | 12-09-1989 |

EPO FORM P0459

For more details about this annex : see Official Journal of the European Patent Office, No. 12/82